# EUROPEAN PATENT APPLICATION

(11) **EP 4 079 388 A1**
(43) Date of publication of application: **26.10.2022**
(21) Application number: 20903092.3
(22) Date of filing: 30.11.2020
(51) Int. Cl.: B01D 19/04, G01N 33/48

(54) **BLOOD COLLECTION CONTAINER**

(30) Priority: 18.12.2019 JP 2019228309
(71) Applicant: Sekisui Medical Co., Ltd., Tokyo 103-0027 (JP); Tokuyama Sekisui Co., Ltd., Osaka-Shi, Osaka 530-8565 (JP)
(72) Inventor: SETOGUCHI, Yuji, Shunan-shi, Yamaguchi 746-0006 (JP); ISOGAWA, Hironobu, Shunan-shi, Yamaguchi 746-0006 (JP)
(74) Representative: Ter Meer Steinmeister & Partner
(86) International application number: PCT/JP2020/044441
(87) International publication number: WO 2021/124847

(57) **Abstract**

Provide is a blood collection container capable of suppressing the occurrence of blood clots involving bubbles when blood containing heparin is coagulated, and capable of suppressing the production of fibrin in serum after separation when blood containing heparin is separated into serum and blood clots. A blood collection container according to the present invention includes: a blood collection container main body having an opening at one end thereof and a closed bottom at the other end thereof; a serine protease disposed in the blood collection container main body; and a heparin neutralizing agent disposed in the blood collection container main body, wherein when a region in which the serine protease is disposed is defined as a first region, and a region in which the heparin neutralizing agent is disposed is defined as a second region, the second region includes a region present on an other end side with respect to an end on the other end side of the first region.

## Description

### TECHNICAL FIELD

The present invention relates to a blood collection container.

### BACKGROUND ART

In clinical examinations, blood collection containers such as blood collection tubes are widely used for collecting blood samples. Blood is collected in a blood collection container containing a serum separating composition, and the blood is coagulated and then centrifuged, whereby the blood can be separated into serum and blood clots. At this time, the serum is located above the serum separating composition, and the blood clots are located below the serum separating composition.

As an example of the blood collection container, Patent Document 1 below discloses a blood collection container wherein a blood coagulation accelerator and an antifoaming agent that is polyoxyalkylene or a derivative thereof are housed, and the amount of the antifoaming agent is 2.0 × 10⁻³ mg to 0.2 mg per 1 mL of blood collected in the blood collection container.

In addition, Patent Document 2 below discloses a blood test container including a bottomed tubular container in which a blood coagulation accelerator is present. The blood coagulation accelerator contains thrombin or thrombin-like serine protease as a blood coagulation accelerating component, contains an amine salt and/or an organic compound having quaternary nitrogen as a heparin neutralizing component, and contains an antifibrinolytic agent or an antiplasmin agent as a clot stabilization component. Patent Document 2 indicates that even blood containing heparin can be coagulated in a short time when the above-described blood test container is used.

### Related Art Document

### Patent Document

Patent Document 1: WO 2010/024325 A1
Patent Document 2: JP H8-154697 A

### SUMMARY OF THE INVENTION

### PROBLEMS TO BE SOLVED BY THE INVENTION

Blood of a patient to which heparin is administered, such as a dialysis patient or a thrombosis patient, is less likely to coagulate than normal blood. For this reason, when blood containing heparin is to be separated into serum and blood clots using a conventional blood collection container as described in Patent Document 1, the blood cannot be coagulated in a short time, or the blood cannot be satisfactorily separated into serum and blood clots in some cases.

On the other hand, Patent Document 2 discloses a blood collection container in which a heparin neutralizing agent and a blood coagulation accelerating component (serine protease such as thrombin) are arranged at the same position. When a blood collection container in which a heparin neutralizing agent is disposed is used, blood containing heparin can be coagulated in a short time, and blood containing heparin can be separated into serum and blood clots with a certain degree of success.

However, in the blood collection container disclosed in Patent Document 2, when blood containing heparin is coagulated, blood clots involving bubbles (so-called bubble-involving fibrin) may occur. When blood clots involving bubbles occur, the clots float on the serum side at the time of centrifugation, and red blood cells and the like are mixed in the serum, which may affect the test result of the serum.

In the blood collection container described in Patent Document 2, when blood containing heparin is separated into serum and blood clots using a serum separating composition, fibrin may be produced in the serum after the separation in some cases. The production of fibrin in serum after separation (so-called delayed fibrin) is due to poor coagulation, and may again affect the test result of the serum in some cases.

It is an object of the present invention to provide a blood collection container capable of suppressing the occurrence of blood clots involving bubbles when blood containing heparin is coagulated, and capable of suppressing the production of fibrin in serum after separation when blood containing heparin is separated into serum and blood clots.

### MEANS FOR SOLVING THE PROBLEM

According to a broad aspect of the present invention, a blood collection container is provided that includes: a blood collection container main body having an opening at one end thereof and a closed bottom at the other end thereof; a serine protease disposed in the blood collection container main body; and a heparin neutralizing agent disposed in the blood collection container main body, wherein when a region in which the serine protease is disposed is defined as a first region, and a region in which the heparin neutralizing agent is disposed is defined as a second region, the second region includes a region present on a side of the other end of the blood collection container main body (on the other end side) with respect to an end on the other end side of the first region.

In a specific aspect of the blood collection container according to the present invention, there is, or there is not an overlapping region in which the first region and the second region overlap, wherein when there is an overlapping region in which the first region and the second region overlap, the overlapping region having an end on a side of the one end of the blood collection container main body (the one end side) and an end on the other end side, a ratio of a distance between the end on the one end side and the end on the other end side to a distance between the end on the one end side and the end on the other end side of the second region is 0.2 or less.

In another specific aspect of the blood collection container according to the present invention, the blood collection container further includes an inorganic powder disposed in the blood collection container main body, wherein when a region in which the inorganic powder is disposed is defined as a third region, the third region includes a region present on the other end side with respect to an end on the other end side of the first region.

In still another specific aspect of the blood collection container according to the present invention, the third region includes a region present on the one end side with respect to an end on the one end side of the second region.

In still another specific aspect of the blood collection container according to the present invention, the second region includes a region present on the other end side with respect to an end on the other end side of the third region.

In still another specific aspect of the blood collection container according to the present invention, the inorganic powder is silica powder.

In still another specific aspect of the blood collection container according to the present invention, the serine protease is thrombin, a thrombin-like enzyme, or a fibrinogen degrading enzyme.

In still another specific aspect of the blood collection container according to the present invention, the blood collection container includes an antifoaming agent disposed at least at the bottom in the blood collection container main body.

In still another specific aspect of the blood collection container according to the present invention, the blood collection container includes a serum separating composition housed at the bottom in the blood collection container main body.

### EFFECT OF THE INVENTION

A blood collection container according to the present invention includes a blood collection container main body having an opening at one end thereof and a closed bottom at the other end thereof, a serine protease disposed in the blood collection container main body, and a heparin neutralizing agent disposed in the blood collection container main body. In the present invention, a region in which the serine protease is disposed is defined as a first region, and a region in which the heparin neutralizing agent is disposed is defined as a second region. In the blood collection container according to the present invention, the second region includes a region present on the other end side with respect to an end on the other end side of the first region. Since the blood collection container according to the present invention is provided with the above-described configuration, it is possible to suppress the occurrence of blood clots involving bubbles when blood containing heparin is coagulated, and it is possible to suppress the production of fibrin in serum after separation when blood containing heparin is separated into serum and blood clots.

### BRIEF DESCRIPTION OF DRAWINGS

[Fig. 1] Fig. 1 is a front cross-sectional view of a blood collection container according to a first embodiment of the present invention.
[Fig. 2] Fig. 2 is a front cross-sectional view of a blood collection container according to a second embodiment of the present invention.
[Fig. 3] Fig. 3 is a front cross-sectional view of a blood collection container according to a third embodiment of the present invention.
[Fig. 4] Fig. 4 is a front cross-sectional view of a blood collection container according to a fourth embodiment of the present invention.
[Fig. 5] Fig. 5 is a front cross-sectional view of a blood collection container according to a fifth embodiment of the present invention.
[Fig. 6] Fig. 6 is a front cross-sectional view of a blood collection container according to a sixth embodiment of the present invention.
[Fig. 7] Fig. 7 is a front cross-sectional view of a blood collection container according to a seventh embodiment of the present invention.

### MODE(S) FOR CARRYING OUT THE INVENTION

Hereinafter, the present invention will be described in detail.

A blood collection container according to the present invention includes a blood collection container main body having an opening at one end thereof and a closed bottom at the other end thereof, a serine protease disposed in the blood collection container main body, and a heparin neutralizing agent disposed in the blood collection container main body.

In the present invention, a region in which the serine protease is disposed is defined as a first region, and a region in which the heparin neutralizing agent is disposed is defined as a second region. In the blood collection container according to the present invention, the second region includes a region present on the other end side with respect to an end on the other end side of the first region.

Since the blood collection container according to the present invention is provided with the above-described configuration, it is possible to suppress the occurrence of blood clots involving bubbles when blood containing heparin is coagulated, and it is possible to suppress the production of fibrin in serum after separation when blood containing heparin is separated into serum and blood clots. In the blood collection container according to the present invention, blood containing heparin can be coagulated in a short time. In the blood collection container according to the present invention, for example, blood containing one unit of heparin can be coagulated in 5 minutes to 10 minutes.

In a conventional blood collection container containing a heparin neutralizing agent, the heparin neutralizing agent and a blood coagulation accelerating component (serine protease such as thrombin) are applied over an entire inner surface of the blood collection container main body. The present inventors have found that when blood containing heparin is collected in this blood collection container, a blood coagulation reaction proceeds at the same time as the blood collection, blood clots involving bubbles such as air bubbles formed at the time of the blood collection occur, and coagulation failure occurs, whereby fibrin is produced in serum after the separation.

Furthermore, the present inventors have found that when blood containing heparin is collected in a blood collection container in which a mixture of a heparin neutralizing agent and a blood coagulation accelerating component (serine protease such as thrombin) is arranged in a narrow range near the opening of the blood collection container main body, the following tends to occur. That is, the present inventors have found that when blood containing heparin is collected in this blood collection container, the inactivation reaction of thrombin is dramatically promoted by the heparin-antithrombin III complex contained in the blood, and the occurrence of blood clots involving bubbles and the production of fibrin in serum after separation are more likely to occur.

On the other hand, in the blood collection container according to the present invention, the region (second region) in which the heparin neutralizing agent is disposed includes a portion arranged on a lower side (the side of the other end of the blood collection container main body, i.e., the other end side) with respect to the region (first region) in which the serine protease is disposed. Therefore, when blood containing heparin is collected using the blood collection container according to the present invention, the blood comes into contact with the heparin neutralizing agent before blood comes into contact with the serine protease, so that the heparin neutralizing agent neutralizes heparin in the blood, and the formation of the heparin-antithrombin III complex can be effectively suppressed. Subsequently, inversion mixing or the like is performed, whereby the blood and the serine protease come into contact with each other, which causes the blood coagulation reaction to effectively proceed, causing the blood to be coagulated in a short time. In addition, when an inorganic powder is disposed in the blood collection container main body, the blood coagulation reaction can be more effectively advanced. Therefore, it is possible to suppress the occurrence of blood clots involving bubbles and the production of fibrin in serum after separation.

Hereinafter, specific embodiments of the present invention are described with reference to the drawings.

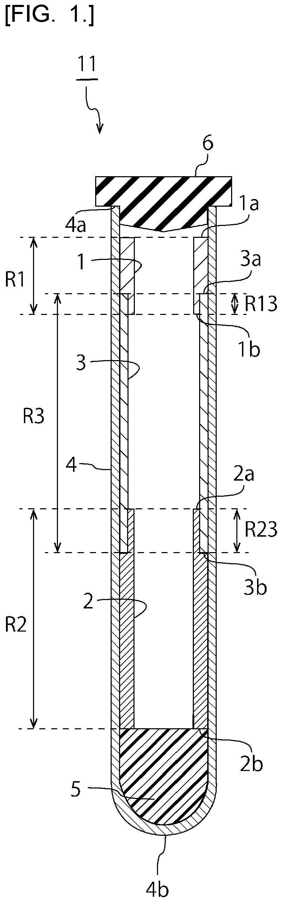

Fig. 1 is a front cross-sectional view of a blood collection container according to a first embodiment of the present invention.

A blood collection container 11 includes a serine protease 1 (a serine protease containing layer), a heparin neutralizing agent 2 (a heparin neutralizing agent containing layer), an inorganic powder 3 (an inorganic powder containing layer), a blood collection container main body 4, a serum separating composition 5, and a plug 6. The blood collection container main body 4 has an opening at one end 4a thereof and a closed bottom at the other end 4b thereof. The serum separating composition 5 is housed in a bottom portion of the blood collection container main body 4. The plug 6 is inserted into the opening of the blood collection container main body 4. In addition, the blood collection container 11 includes an antifoaming agent (not illustrated) disposed at least in a bottom portion of the blood collection container main body 4. The blood collection container 11 is a vacuum blood collection tube.

Each of the serine protease 1, the heparin neutralizing agent 2, the inorganic powder 3, and the antifoaming agent is disposed in the blood collection container main body 4, attached to an inner wall surface of the blood collection container main body 4, and disposed on the inner wall surface of the blood collection container main body 4.

A first region R1 is a region in which the serine protease 1 is disposed. The first region R1 is an annular region.

A second region R2 is a region in which the heparin neutralizing agent 2 is disposed. The second region R2 is an annular region.

A third region R3 is a region where the inorganic powder 3 is disposed. The third region R3 is an annular region.

In the blood collection container 11, the second region R2 includes a region present on the other end 4b side with respect to an end 1b on the other end 4b side of the first region R1. In the blood collection container 11, the entire second region R2 is present on the other end 4b side with respect to the end 1b on the other end 4b side of the first region R1. The second region R2 has an end 2a on the one end 4a side thereof, the end 2a being located on the other end 4b side with respect to the end 1b on the other end 4b side of the first region R1.

Therefore, in the blood collection container 11, there is no overlapping region where the first region R1 and the second region R2 overlap.

In the blood collection container 11, the third region R3 includes a region present on the other end 4b side with respect to the end 1b on the other end 4b side of the first region R1. The third region R3 has an end 3b on the other end 4b side thereof, the end 3b being located on the other end 4b side with respect to the end 1b on the other end 4b side of the first region R1.

In the blood collection container 11, the first region R1 includes a region present on the one end 4a side with respect to an end 3a on the one end 4a side of the third region R3. The first region R1 has an end 1a on the one end 4a side thereof, the end 1a being located on the one end 4a side with respect to the end 3a on the one end 4a side of the third region R3. Incidentally, the end on the one end side of the first region R1 and the end on the one end side of the third region R3 may be extended to the vicinity of the one end of the blood collection container main body, or may be at the same position in the vicinity of the one end of the blood collection container main body.

In the blood collection container 11, the end 1b on the other end 4b side of the first region R1 is located on the other end 4b side with respect to the end 3a on the one end 4a side of the third region R3. Therefore, in the blood collection container 11, there is an overlapping region R13 in which the first region R1 and the third region R3 overlap. In the blood collection container 11, the overlapping region R13 is an annular region. In the blood collection container 11, in the overlapping region R13, the serine protease containing layer is located on an inner side with respect to the inorganic powder containing layer.

In the overlapping region R13, the serine protease and the inorganic powder are present in a mixed state.

In the blood collection container 11, the third region R3 includes a region present on the one end 4a side with respect to an end 2a on the one end 4a side of the second region R2. The end 3a on the one end 4a side of the third region R3 is located on the one end 4a side with respect to the end 2a on the one end 4a side of the second region R2.

In the blood collection container 11, the second region R2 includes a region present on the other end 4b side with respect to the end 3b on the other end 4b side of the third region R3. The second region R2 includes an end 2b on the other end 4b side, the end 2b being located on the other end 4b side with respect to the end 3b on the other end 4b side of the third region R3.

In the blood collection container 11, the end 2a on the one end 4a side of the second region R2 is located on the one end 4a side with respect to the end 3b on the other end 4b side of the third region R3. Therefore, in the blood collection container 11, there is an overlapping region R23 in which the second region R2 and the third region R3 overlap. In the blood collection container 11, the overlapping region R23 is an annular region. In the blood collection container 11, in the overlapping region R23, the heparin neutralizing agent containing layer is located on an inner side with respect to the inorganic powder containing layer.

In the overlapping region R23, the heparin neutralizing agent and the inorganic powder are present in a mixed state.

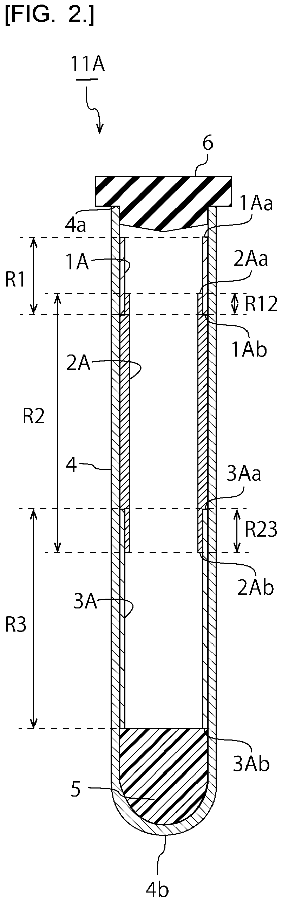

Fig. 2 is a front cross-sectional view of a blood collection container according to a second embodiment of the present invention.

The blood collection container 11A includes a serine protease 1A (a serine protease containing layer), a heparin neutralizing agent 2A (a heparin neutralizing agent containing layer), an inorganic powder 3A (an inorganic powder containing layer), a blood collection container main body 4, a serum separating composition 5, and a plug 6. The blood collection container main body 4 has an opening at one end 4a thereof and a closed bottom at the other end 4b thereof. The serum separating composition 5 is housed in a bottom portion of the blood collection container main body 4. The plug 6 is inserted into the opening of the blood collection container main body 4. In addition, the blood collection container 11A includes an antifoaming agent (not illustrated) disposed at least at the bottom of the blood collection container main body 4. The blood collection container 11A is in a tubular shape, and is a vacuum blood collection tube.

Each of the serine protease 1A, the heparin neutralizing agent 2A, the inorganic powder 3A, and the antifoaming agent is disposed in the blood collection container main body 4, attached to an inner wall surface of the blood collection container main body 4, and disposed on the inner wall surface of the blood collection container main body 4.

A first region R1 is a region in which the serine protease 1A is disposed. The first region R1 is an annular region.

A second region R2 is a region in which the heparin neutralizing agent 2A is disposed. The second region R2 is an annular region.

A third region R3 is a region where the inorganic powder 3A is disposed. The third region R3 is an annular region.

In the blood collection container 11A, the second region R2 includes a region present on the other end 4b side with respect to an end 1Ab on the other end 4b side of the first region R1. The second region R2 includes an end 2Ab on the other end 4b side thereof, the end 2Ab being located on the other end 4b side with respect to the end 1Ab on the other end 4b side of the first region R1.

In the blood collection container 11A, the first region R1 includes a region present on the one end 4a side with respect to the end 2Aa on the one end 4a side of the second region R2. The first region R1 includes an end 1Aa on the one end 4a side thereof, the end 1Aa being located on the one end 4a side with respect to the end 2Aa on the one end 4a side of the second region R2.

In the blood collection container 11A, the end 1Ab on the other end 4b side of the first region R1 is located on the other end 4b side with respect to the end 2Aa on the one end 4a side of the second region R2. Therefore, in the blood collection container 11A, there is an overlapping region R12 in which the first region R1 and the second region R2 overlap. In the blood collection container 11A, the overlapping region R12 is an annular region. In the blood collection container 11A, in the overlapping region R12, the heparin neutralizing agent containing layer is located on an inner side with respect to the serine protease containing layer.

In the overlapping region R12, a serine protease and a heparin neutralizing agent are present in a mixed state.

In the present invention, it is preferable that the overlapping region R12 where the first region R1 and the second region R2 overlap is extremely small, and it is more preferable that there is no overlapping region. In this case, the serine protease can be brought into contact with the blood after the heparin in the blood is neutralized with the heparin neutralizing agent, whereby the blood can be sufficiently coagulated.

In the blood collection container 11A, the third region R3 includes a region present on the other end 4b side with respect to the end 1Ab on the other end 4b side of the first region R1. In the blood collection container 11A, the entire third region R3 exists on the other end 4b side of the end 1Ab on the other end 4b side of the first region R1. The third region R3 has an end 3Aa on the one end 4a side thereof, the end 3Aa being located on the other end 4b side with respect to the end 1Ab on the other end 4b side of the first region R1.

Therefore, in the blood collection container 11A, there is no overlapping region where the first region R1 and the third region R3 overlap.

In the blood collection container 11A, the third region R3 includes a region present on the other end 4b side with respect to the end 2Ab on the other end 4b side of the second region R2. The third region R3 includes an end 3Ab on the other end 4b side thereof, the end 3Ab being located on the other end 4b side with respect to the end 2Ab on the other end 4b side of the second region R2.

In the blood collection container 11A, the second region R2 includes a region present on the one end 4a side with respect to the end 3Aa on the one end 4a side of the third region R3. The second region R2 includes an end 2Aa on the one end 4a side thereof, the end 2Aa being located on the one end 4a side with respect to the end 3Aa on the one end 4a side of the third region R3.

In the blood collection container 11A, the end 2Ab on the other end 4b side of the second region R2 is located on the other end 4b side with respect to the end 3Aa on the one end 4a side of the third region R3. Therefore, in the blood collection container 11A, there is an overlapping region R23 in which the second region R2 and the third region R3 overlap. In the blood collection container 11A, the overlapping region R23 is an annular region. In the blood collection container 11A, in the overlapping region R23, the heparin neutralizing agent containing layer is located on an inner side with respect to the inorganic powder containing layer.

In the overlapping region R23, the heparin neutralizing agent and the inorganic powder are present in a mixed state.

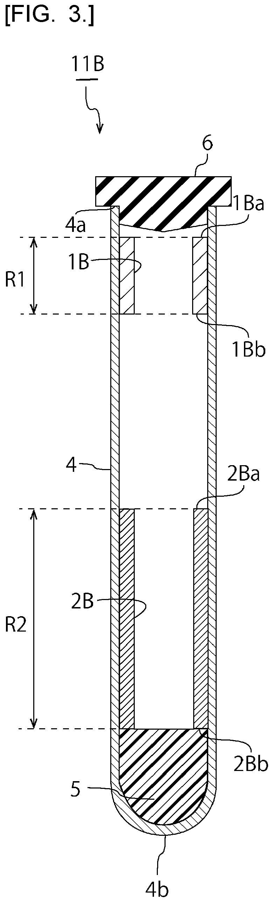

Fig. 3 is a front cross-sectional view of a blood collection container according to a third embodiment of the present invention.

A blood collection container 11B includes a serine protease 1B (a serine protease containing layer), a heparin neutralizing agent 2B (a heparin neutralizing agent containing layer), a blood collection container main body 4, a serum separating composition 5, and a plug 6. Unlike the blood collection container 11 shown in Fig. 1, the blood collection container 11B does not include an inorganic powder. The blood collection container main body 4 has an opening at one end 4a thereof and a closed bottom at the other end 4b thereof. The serum separating composition 5 is housed in a bottom portion of the blood collection container main body 4. The plug 6 is inserted into the opening of the blood collection container main body 4. In addition, the blood collection container 11B includes an antifoaming agent (not illustrated) disposed at least at the bottom of the blood collection container main body 4. The blood collection container 11B is in a tubular shape, and is a vacuum blood collection tube.

Each of the serine protease 1B, the heparin neutralizing agent 2B, and the antifoaming agent is disposed in the blood collection container main body 4, attached to the inner wall surface of the blood collection container main body 4, and disposed on the inner wall surface of the blood collection container main body 4.

A first region R1 is a region in which serine protease 1B is disposed. The first region R1 is an annular region.

A second region R2 is a region in which the heparin neutralizing agent 2B is disposed. The second region R2 is an annular region.

In the blood collection container 11B, the second region R2 includes a region present on the other end 4b side with respect to the end 1Bb on the other end 4b side of the first region R1. In the blood collection container 11B, the entire second region R2 is present on the other end 4b side with respect to the end 1Bb on the other end 4b side of the first region R1. The second region R2 has an end 2Ba on the one end 4a side thereof, the end 2Ba being located on the other end 4b side with respect to the end 1Bb on the other end 4b side of the first region R1. Incidentally, the reference numeral "1Ba" denotes an end on the one end 4a side of the first region R1, and the reference numeral "2Bb" denotes an end on the other end 4b side of the second region R2.

Therefore, in the blood collection container 11B, there is no overlapping region where the first region R1 and the second region R2 overlap. Incidentally, in a blood collection container not including the third region R3, unlike the blood collection container 11B illustrated in Fig. 3, there may be an overlapping region R12 in which the first region R1 and the second region R2 overlap, but it is preferable that the overlapping region R12 is as small as possible.

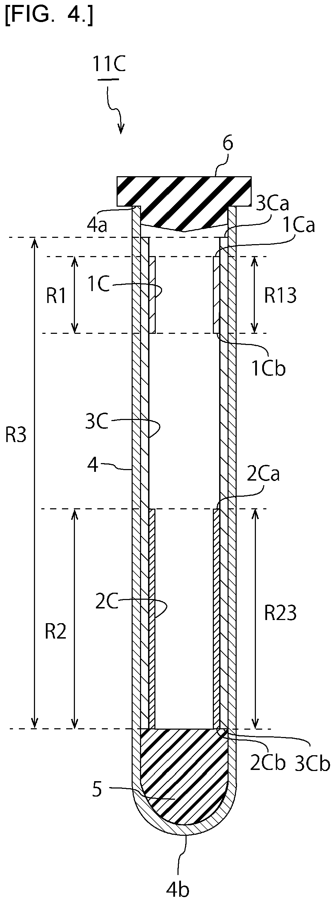

Fig. 4 is a front cross-sectional view of a blood collection container according to a fourth embodiment of the present invention.

The blood collection container 11C includes a serine protease 1C (a serine protease containing layer), a heparin neutralizing agent 2C (a heparin neutralizing agent containing layer), an inorganic powder 3C (an inorganic powder containing layer), a blood collection container main body 4, a serum separating composition 5, and a plug 6. The blood collection container main body 4 has an opening at one end 4a thereof and a closed bottom at the other end 4b thereof. The serum separating composition 5 is housed in a bottom portion of the blood collection container main body 4. The plug 6 is inserted into the opening of the blood collection container main body 4. In addition, the blood collection container 11C includes an antifoaming agent (not illustrated) disposed at least at the bottom of the blood collection container main body 4. The blood collection container 11C is in a tubular shape, and is a vacuum blood collection tube.

Each of the serine protease 1C, the heparin neutralizing agent 2C, the inorganic powder 3C, and the antifoaming agent is disposed in the blood collection container main body 4, attached to an inner wall surface of the blood collection container main body 4, and disposed on the inner wall surface of the blood collection container main body 4.

A first region R1 is a region in which serine protease 1C is disposed. The first region R1 is an annular region.

A second region R2 is a region in which the heparin neutralizing agent 2C is disposed. The second region R2 is an annular region.

A third region R3 is a region where the inorganic powder 3C is disposed. The third region R3 is an annular region.

In the blood collection container 11C, the second region R2 includes a region present on the other end 4b side with respect to the end 1Cb on the other end 4b side of the first region R1. In the blood collection container 11C, the entire second region R2 is present on the other end 4b side with respect to the end 1Cb on the other end 4b side of the first region R1. The second region R2 has an end 2Ca on the one end 4a side thereof, the end 2Ca being located on the other end 4b side with respect to the end 1Cb on the other end 4b side of the first region R1.

Therefore, in the blood collection container 11C, there is no overlapping region where the first region R1 and the second region R2 overlap.

In the blood collection container 11C, the third region R3 includes a region present on the one end 4a side with respect to the end 1Ca on the one end 4a side of the first region R1. The third region R3 includes an end 3Ca on the one end 4a side thereof, the end 3Ca being located on the one end 4a side with respect to the end 1Ca on the one end 4a side of the first region R1.

In the blood collection container 11C, the third region R3 includes a region present on the other end 4b side with respect to the end 1Cb on the other end 4b side of the first region R1. The third region R3 includes an end 3Cb on the other end 4b side thereof, the end 3Cb being located on the other end 4b side with respect to the end 1Cb on the other end 4b side of the first region R1.

Therefore, in the blood collection container 11C, there is an overlapping region R13 in which the first region R1 and the third region R3 overlap. In the blood collection container 11C, the overlapping region R13 coincides with the first region R1. In the blood collection container 11C, the overlapping region R13 is an annular region. In the blood collection container 11C, in the overlapping region R13, the serine protease containing layer is located on an inner side with respect to the inorganic powder containing layer.

In the overlapping region R13, the serine protease and the inorganic powder are present in a mixed state.

In the blood collection container 11C, the third region R3 includes a region present on the one end 4a side with respect to the end 2Ca on the one end 4a side of the second region R2. The third region R3 includes an end 3Ca on the one end 4a side thereof, the end 3Ca being located on the one end 4a side with respect to the end 2Ca on the one end 4a side of the second region R2.

In the blood collection container 11C, the end 2Cb on the other end 4b side of the second region R2 and the end 3Cb on the other end 4b side of the third region R3 are present at the same position.

Therefore, in the blood collection container 11C, there is an overlapping region R23 in which the second region R2 and the third region R3 overlap. In the blood collection container 11C, the overlapping region R23 coincides with the second region R2. In the blood collection container 11C, the overlapping region R23 is an annular region. In the blood collection container 11C, in the overlapping region R23, the heparin neutralizing agent containing layer is located on an inner side with respect to the inorganic powder containing layer.

In the overlapping region R23, the heparin neutralizing agent and the inorganic powder are present in a mixed state.

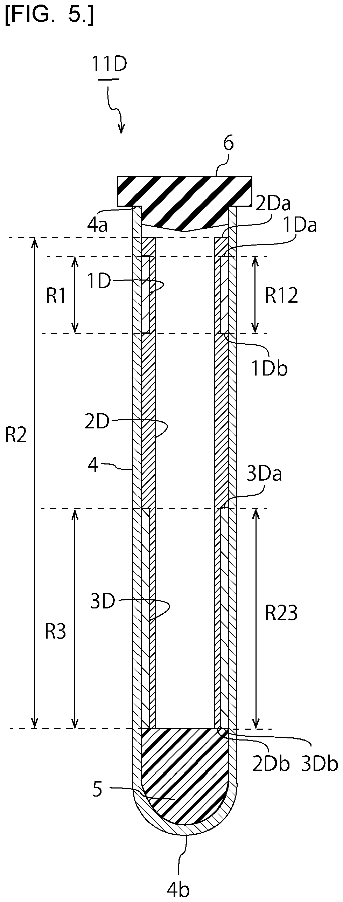

Fig. 5 is a front cross-sectional view of a blood collection container according to a fifth embodiment of the present invention.

The blood collection container 11D includes a serine protease 1D (a serine protease containing layer), a heparin neutralizing agent 2D (a heparin neutralizing agent containing layer), an inorganic powder 3D (an inorganic powder containing layer), a blood collection container main body 4, a serum separating composition 5, and a plug 6. The blood collection container main body 4 has an opening at one end 4a thereof and a closed bottom at the other end 4b thereof. The serum separating composition 5 is housed in a bottom portion of the blood collection container main body 4. The plug 6 is inserted into the opening of the blood collection container main body 4. In addition, the blood collection container 11D includes an antifoaming agent (not illustrated) disposed at least at the bottom of the blood collection container main body 4. The blood collection container 11D is in a tubular shape, and is a vacuum blood collection tube.

Each of the serine protease 1D, the heparin neutralizing agent 2D, the inorganic powder 3D, and the antifoaming agent is disposed in the blood collection container main body 4, attached to an inner wall surface of the blood collection container main body 4, and disposed on the inner wall surface of the blood collection container main body 4.

A first region R1 is a region in which serine protease 1D is disposed. The first region R1 is an annular region.

A second region R2 is a region in which the heparin neutralizing agent 2D is disposed. The second region R2 is an annular region.

A third region R3 is a region where the inorganic powder 3D is disposed. The third region R3 is an annular region.

In the blood collection container 11D, the second region R2 includes a region present on the other end 4b side with respect to the end 1Db on the other end 4b side of the first region R1. The second region R2 includes an end 2Db on the other end 4b side thereof, the end 2Db being located on the other end 4b side with respect to the end 1Db on the other end 4b side of the first region R1.

In the blood collection container 11D, the second region R2 includes a region present on the one end 4a side with respect to the end 1Da on the one end 4a side of the first region R1. The second region R2 includes an end 2Da on the one end 4a side thereof, the end 2Da being located on the one end 4a side with respect to the end 1Da on the one end 4a side of the first region R1.

Therefore, in the blood collection container 11D, there is an overlapping region R12 in which the first region R1 and the second region R2 overlap. In the blood collection container 11D, the overlapping region R12 coincides with the first region R1. In the blood collection container 11D, the overlapping region R12 is an annular region. In the blood collection container 11D, in the overlapping region R12, the heparin neutralizing agent containing layer is located on an inner side with respect to the serine protease containing layer.

In the overlapping region R12, a serine protease and a heparin neutralizing agent are present in a mixed state.

In the blood collection container 11D, the second region R2 includes a region present on the one end 4a side with respect to the end 3Da on the one end 4a side of the third region R3. The second region R2 includes an end 2Da on the one end 4a side thereof, the end 2Da being located on the one end 4a side with respect to the end 3Da on the one end 4a side of the third region R3.

In the blood collection container 11D, the end 2Db on the other end 4b side of the second region R2 and the end 3Db on the other end 4b side of the third region R3 are present at the same position.

Therefore, in the blood collection container 11D, there is an overlapping region R23 in which the second region R2 and the third region R3 overlap. In the blood collection container 11D, the overlapping region R23 coincides with the third region R3. In the blood collection container 11D, the overlapping region R23 is an annular region. In the blood collection container 11D, in the overlapping region R23, the heparin neutralizing agent containing layer is located on an inner side with respect to the inorganic powder containing layer.

In the overlapping region R23, the heparin neutralizing agent and the inorganic powder are present in a mixed state.

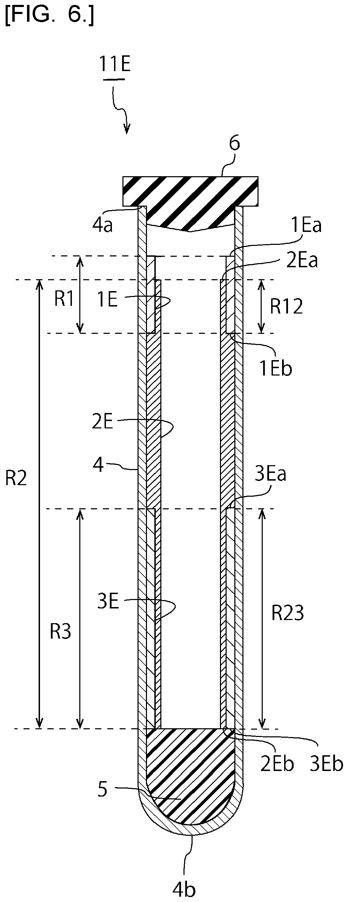

Fig. 6 is a front cross-sectional view of a blood collection container according to a sixth embodiment of the present invention.

The blood collection container 11E includes a serine protease 1E (a serine protease containing layer), a heparin neutralizing agent 2E (a heparin neutralizing agent containing layer), an inorganic powder 3E (an inorganic powder containing layer), a blood collection container main body 4, a serum separating composition 5, and a plug 6. The blood collection container main body 4 has an opening at one end 4a thereof and a closed bottom at the other end 4b thereof. The serum separating composition 5 is housed in a bottom portion of the blood collection container main body 4. The plug 6 is inserted into the opening of the blood collection container main body 4. In addition, the blood collection container 11E includes an antifoaming agent (not illustrated) disposed at least at the bottom of the blood collection container main body 4. The blood collection container 11E is in a tubular shape, and is a vacuum blood collection tube.

Each of the serine protease 1E, the heparin neutralizing agent 2E, the inorganic powder 3E, and the antifoaming agent is disposed in the blood collection container main body 4, attached to an inner wall surface of the blood collection container main body 4, and disposed on the inner wall surface of the blood collection container main body 4.

A first region R1 is a region in which serine protease 1E is disposed. The first region R1 is an annular region.

A second region R2 is a region in which the heparin neutralizing agent 2E is disposed. The second region R2 is an annular region.

A third region R3 is a region where the inorganic powder 3E is disposed. The third region R3 is an annular region.

In the blood collection container 11E, the second region R2 includes a region present on the other end 4b side with respect to the end 1Eb on the other end 4b side of the first region R1. The second region R2 includes an end 2Eb on the other end 4b side thereof, the end 2Eb being located on the other end 4b side with respect to the end 1Eb on the other end 4b side of the first region R1.

In the blood collection container 11E, the first region R1 includes a region present on the one end 4a side with respect to the end 2Ea on the one end 4a side of the second region R2. The first region R1 includes an end 1Ea on the one end 4a side thereof, the end 1Ea being located on the one end 4a side with respect to the end 2Ea on the one end 4a side of the second region R2.

Therefore, in the blood collection container 11E, there is an overlapping region R12 in which the first region R1 and the second region R2 overlap. In the blood collection container 11E, the overlapping region R12 is an annular region. In the blood collection container 11E, in the overlapping region R12, the heparin neutralizing agent containing layer is located on an inner side with respect to the serine protease containing layer.

In the overlapping region R12, a serine protease and a heparin neutralizing agent are present in a mixed state.

In the blood collection container 11E, the second region R2 includes a region present on the one end 4a side with respect to the end 3Ea on the one end 4a side of the third region R3. The second region R2 includes an end 2Ea on the one end 4a side thereof, the end 2Ea being located on the one end 4a side with respect to the end 3Ea on the one end 4a side of the third region R3.

In the blood collection container 11E, the end 2Eb on the other end 4b side of the second region R2 and the end 3Eb on the other end 4b side of the third region R3 are present at the same position.

Therefore, in the blood collection container 11E, there is an overlapping region R23 in which the second region R2 and the third region R3 overlap. In the blood collection container 11E, the overlapping region R23 coincides with the third region R3. In the blood collection container 11E, the overlapping region R23 is an annular region. In the blood collection container 11E, in the overlapping region R23, the heparin neutralizing agent containing layer is located on an inner side with respect to the inorganic powder containing layer.

In the overlapping region R23, the heparin neutralizing agent and the inorganic powder are present in a mixed state.

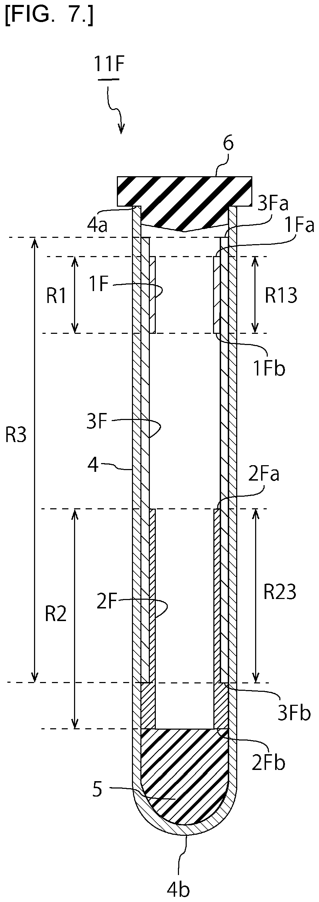

Fig. 7 is a front cross-sectional view of a blood collection container according to a seventh embodiment of the present invention.

The blood collection container 11F includes a serine protease 1F (a serine protease containing layer), a heparin neutralizing agent 2F (a heparin neutralizing agent containing layer), an inorganic powder 3F (an inorganic powder containing layer), a blood collection container main body 4, a serum separating composition 5, and a plug 6. The blood collection container main body 4 has an opening at one end 4a thereof and a closed bottom at the other end 4b thereof. The serum separating composition 5 is housed in a bottom portion of the blood collection container main body 4. The plug 6 is inserted into the opening of the blood collection container main body 4. In addition, the blood collection container 11F includes an antifoaming agent (not illustrated) disposed at least at the bottom of the blood collection container main body 4. The blood collection container 11F is in a tubular shape, and is a vacuum blood collection tube.

Each of the serine protease 1F, the heparin neutralizing agent 2F, the inorganic powder 3F, and the antifoaming agent is disposed in the blood collection container main body 4, attached to an inner wall surface of the blood collection container main body 4, and disposed on the inner wall surface of the blood collection container main body 4.

A first region R1 is a region in which serine protease 1F is disposed. The first region R1 is an annular region.

A second region R2 is a region in which the heparin neutralizing agent 2F is disposed. The second region R2 is an annular region.

A third region R3 is a region where the inorganic powder 3F is disposed. The third region R3 is an annular region.

In the blood collection container 11F, the second region R2 includes a region present on the other end 4b side with respect to the end 1Fb on the other end 4b side of the first region R1. In the blood collection container 11F, the entire second region R2 is present on the other end 4b side with respect to the end 1Fb on the other end 4b side of the first region R1. The second region R2 has an end 2Fa on the one end 4a side thereof, the end 2Fa being located on the other end 4b side with respect to the end 1Fb on the other end 4b side of the first region R1.

Therefore, in the blood collection container 11F, there is no overlapping region where the first region R1 and the second region R2 overlap.

In the blood collection container 11F, the third region R3 includes a region present on the one end 4a side with respect to the end 1Fa on the one end 4a side of the first region R1. The third region R3 includes an end 3Fa on the one end 4a side thereof, the end 3Fa being located on the one end 4a side with respect to the end 1Fa on the one end 4a side of the first region R1.

In the blood collection container 11F, the third region R3 includes a region present on the other end 4b side with respect to the end 1Fb on the other end 4b side of the first region R1. The third region R3 includes an end 3Fb on the other end 4b side thereof, the end 3Fb being located on the other end 4b side with respect to the end 1Fb on the other end 4b side of the first region R1.

Therefore, in the blood collection container 11F, there is an overlapping region R13 in which the first region R1 and the third region R3 overlap. In the blood collection container 11F, the overlapping region R13 is an annular region. In the blood collection container 11F, in the overlapping region R13, the serine protease containing layer is located on an inner side with respect to the inorganic powder containing layer.

In the overlapping region R13, the serine protease and the inorganic powder are present in a mixed state.

In the blood collection container 11F, the third region R3 includes a region present on the one end 4a side with respect to the end 2Fa on the one end 4a side of the second region R2. The third region R3 includes an end 3Fa on the one end 4a side thereof, the end 3Fa being located on the one end 4a side with respect to the end 2Fa on the one end 4a side of the second region R2.

In the blood collection container 11F, the second region R2 includes a region present on the other end 4b side with respect to the end 3Fb on the other end 4b side of the third region R3. The second region R2 includes an end 2Fb on the other end 4b side, the end 2Fb being located on the other end 4b side with respect to the end 3Fb on the other end 4b side of the third region R3.

Therefore, in the blood collection container 11F, there is an overlapping region R23 in which the second region R2 and the third region R3 overlap. In the blood collection container 11F, the overlapping region R23 is an annular region. In the blood collection container 11F, in the overlapping region R23, the heparin neutralizing agent containing layer is located on an inner side with respect to the inorganic powder containing layer.

In the overlapping region R23, the heparin neutralizing agent and the inorganic powder are present in a mixed state.

In the blood collection container according to the present invention, an end on the one end side (the side of the one end of the blood collection container main body) of the first region R1 may be located on the side of the one end with respect to an end on the one end side (the side of the one end of the blood collection container main body) of the second region R2, or may be located on a side of the other end with respect to the same. In the blood collection container according to the present invention, the end on the one end side of the first region R1 may coincide with the end on the one end side of the second region R2.

In the blood collection container according to the present invention, an end on the one end side (the side of the one end of the blood collection container main body) of the first region R1 may be located on the side of the one end with respect to an end on the one end side (the side of the one end of the blood collection container main body) of the third region R3, or may be located on the side of the other end with respect to the same. In the blood collection container according to the present invention, the end on the one end side of the first region R1 may coincide with the end on the one end side of the third region R3.

In the blood collection container according to the present invention, an end on the one end side (the side of the one end of the blood collection container main body) of the second region R2 may be located on the side of the one end with respect to an end on the one end side (the side of the one end of the blood collection container main body) of the third region R3, or may be located on the side of the other end with respect to the same. In the blood collection container according to the present invention, the end on the one end side of the second region R2 may coincide with the end on the one end side of the third region R3.

In the blood collection container according to the present invention, the end on the other end side (the side of the other end of the blood collection container main body) of the second region R2 may be located on the side of the one end with respect to the end on the other end side (the side of the other end of the blood collection container main body) of the third region R3, or may be located on the side of the other end with respect to the same. In the blood collection container according to the present invention, the end on the side of the other end of the second region R2 may coincide with the end on the side of the other end of the third region R3.

In the blood collection container according to the present invention, each of the first region R1, the second region R2, the third region R3, the overlapping region R12, the overlapping region R23, and the overlapping region R13 may be an annular region, or does not have to be an annular region. The shapes of the first region R1, the second region R2, and the third region R3 are appropriately changed depending on, for example, the shape of the blood collection container main body.

In the blood collection container according to the present invention, in the overlapping region R12, the heparin neutralizing agent containing layer may be located on an inner side with respect to the serine protease containing layer, or may be located on an outer side with respect to the same. In the blood collection container according to the present invention, in the overlapping region R12, the heparin neutralizing agent containing layer is preferably located on an inner side with respect to the serine protease containing layer.

In the blood collection container according to the present invention, in the overlapping region R23, the heparin neutralizing agent containing layer may be located on an inner side with respect to the inorganic powder containing layer, or may be located on an outer side with respect to the same. In the blood collection container according to the present invention, in the overlapping region R23, the heparin neutralizing agent containing layer is preferably located on an inner side with respect to the inorganic powder containing layer. In this case, the amount of the heparin neutralizing agent in contact with blood containing heparin can be increased, and the effect of the present invention can be more effectively exhibited.

In the blood collection container according to the present invention, when blood is collected by, for example, collecting blood or dispensing a blood sample with the other end of the blood collection container being on the lower side, it is preferable that the first region R1 is present on an upper side (on the side of the one end of the blood collection container main body) with respect to the liquid level of the collected blood.

Hereinafter, the blood collection container according to the present invention will be described in more detail.

### (Serine protease: first region R1)

The serine protease is disposed in the blood collection container main body. The serine protease is preferably attached onto the inner wall surface of the blood collection container main body. In this case, the serine protease may be attached on the inner wall surface of the blood collection container main body via, for example, a water-soluble binder as stated below. One kind of the serine protease may be used alone, or two or more kinds thereof may be used in combination. The first region R1 is a region in which a serine protease is disposed.

Examples of the serine protease include thrombin, a thrombin-like enzyme, trypsin, and a fibrinogen degrading enzyme. Examples of the thrombin-like enzyme include snake venom and ecarin.

From the viewpoint of effectively coagulating blood, the serine protease is preferably thrombin, a thrombin-like enzyme, or a fibrinogen degrading enzyme, more preferably thrombin or a thrombin-like enzyme, and still more preferably thrombin. The thrombin-like enzyme is preferably snake venom or ecarin.

In the blood collection container, the content of the serine protease is preferably 0.5 units or more, and more preferably 1 unit or more, as well as preferably 50 units or less, and more preferably 20 units or less, per 1 mL of collected blood. When the content of the serine protease is equal to the above-described lower limit or more than the same, and equal to the above-described upper limit or less than the same, the blood coagulation reaction can be effectively advanced, and the effect of the present invention can be more effectively exhibited.

### (Heparin neutralizing agent: second region R2)

The heparin neutralizing agent is disposed in the blood collection container main body. The heparin neutralizing agent is preferably attached onto the inner wall surface of the blood collection container main body. In this case, the heparin neutralizing agent may be attached on the inner wall surface of the blood collection container main body via, for example, a water-soluble binder or the like as stated below, or may be attached alone on the inner wall surface of the blood collection container main body. One kind of the heparin neutralizing agent may be used alone, or two or more kinds thereof may be used in combination. The second region R2 is a region in which a heparin neutralizing agent is disposed.

Any agent usable as a heparin neutralizing agent can be used as the above-described heparin neutralizing agent, and examples thereof include amine salts and organic compounds having a quaternary nitrogen atom. The amine salt and the organic compound having a quaternary nitrogen atom can adsorb and neutralize heparin in blood to inactivate heparin.

The amine salt may be a primary amine salt, a secondary amine salt, or a tertiary amine salt. The amine constituting the amine salt may be a linear amine, a cyclic amine, a low molecular weight compound, or a high molecular weight compound having a low molecular weight repeating structural unit. The amine constituting the amine salt may be a primary amine, a secondary amine, or a tertiary amine. The acid constituting the amine salt may be an inorganic acid or an organic acid. Examples of the inorganic acid include hydrohalogenic acid such as hydrochloric acid, sulfuric acid, and sulfurous acid, and examples of the organic acid include formic acid and acetic acid. The amine salt may have an acetyl group, an imino group, or an ether group. The amine salt may be an intramolecular salt.

Preferable specific examples of the amine salt include hexadecyldimethylamine hydrochloride represented by the following formula (1) and tetradecyldi(aminoethyl)glycine represented by the following formula (2).

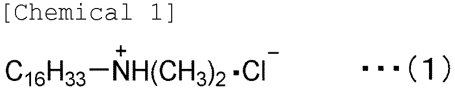

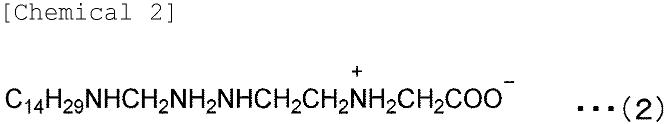

The organic compound having a quaternary nitrogen atom may be a linear compound, a cyclic compound, a low molecular weight compound, or a high molecular weight compound having a low molecular weight repeating structural unit. Examples of the organic compound having a quaternary nitrogen atom include tetraalkylammonium. The organic compound having a quaternary nitrogen atom may have an alkyl group, an aryl group, an imino group, or an ether group.

Preferable specific examples of the organic compound having a quaternary nitrogen atom include dodecyltrimethylammonium chloride represented by the following formula (3).

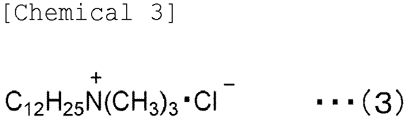

Examples of the organic compound having a quaternary nitrogen atom include a polymer having a quaternary nitrogen, in addition to the compound having a relatively small molecular weight such as those described above. Examples of the polymer having a quaternary nitrogen include a polycation having a repeating structural unit represented by the following formula (4).

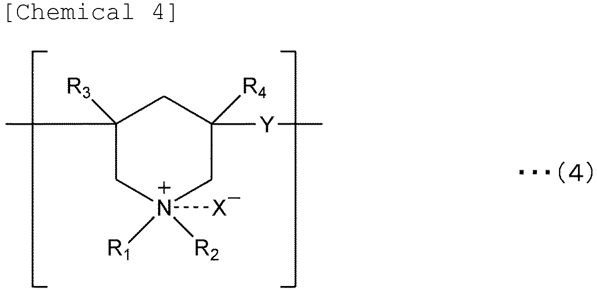

In the above-described formula (4), R₁ to R₄ each represent a hydrogen atom or an alkyl group, X represents a halogen atom or an acid group, and Y represents an alkylene group or a group in which a sulfonyl group is bonded to an alkylene group. In the formula (4), each of R₁ and R₂ is preferably an alkyl group having 5 or less carbon atoms, and is preferably a methyl group or an ethyl group. In the formula (4), each of R₃ and R₄ is preferably a hydrogen atom.

In the polycation having the structural unit represented by the formula (4), the number of repetitions of the structural unit represented by the formula (4) is preferably 5 or more and preferably 2000 or less. Incidentally, the polycation having the structural unit represented by the formula (4) may partially have a structural unit of the formula (4) in which R₁ or R₂ is a hydrogen atom.

The polycation having the structural unit represented by the formula (4) is preferably a polycation having a structural unit represented by the following formula (5) or the following formula (6), and more preferably a polycation having the structure represented by the following formula (6). The polycation having the structure represented by the following formula (6) is polyamine sulfone.

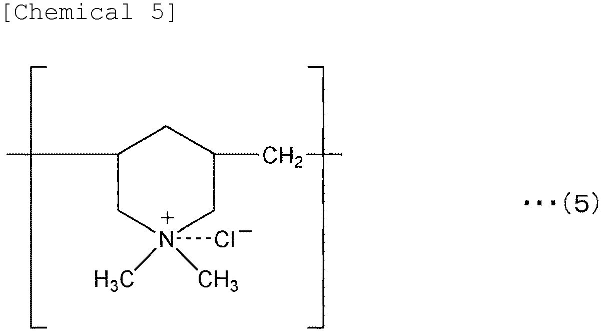

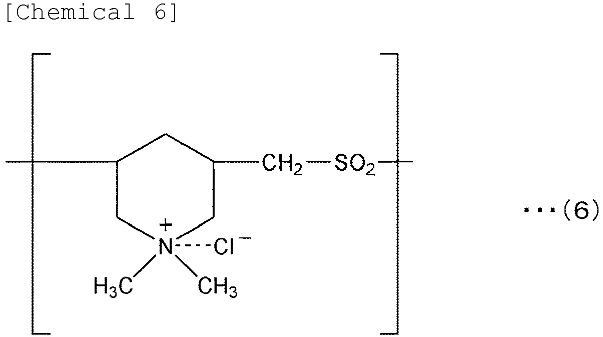

The polycation having the structural unit represented by the formula (5) may be a polycation represented by the following formula (7), a polycation represented by the following formula (8), a polycation represented by the following formula (9), or a polycation represented by the following formula (10). The polycation having the structural unit represented by the formula (4) may be a polycation represented by the formula (5), the formula (6), the formula (7), the formula (8), the formula (9) or the formula (10) in which one or both of two methyl groups are an alkyl group such as an ethyl group.

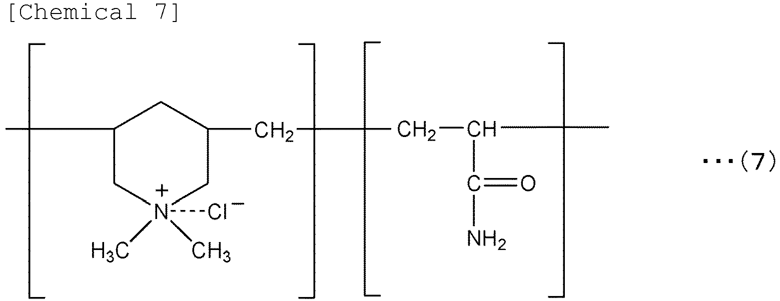

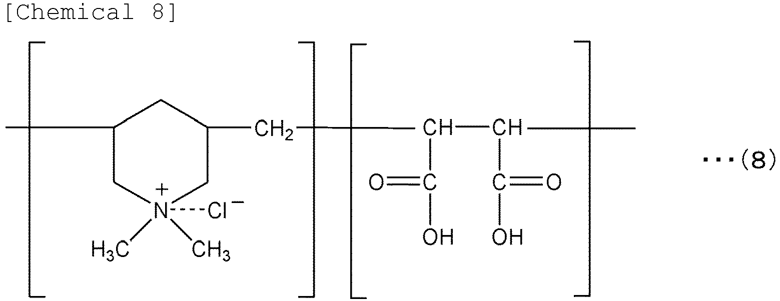

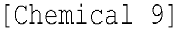

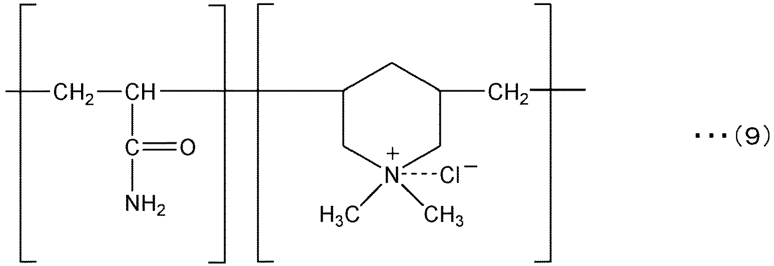

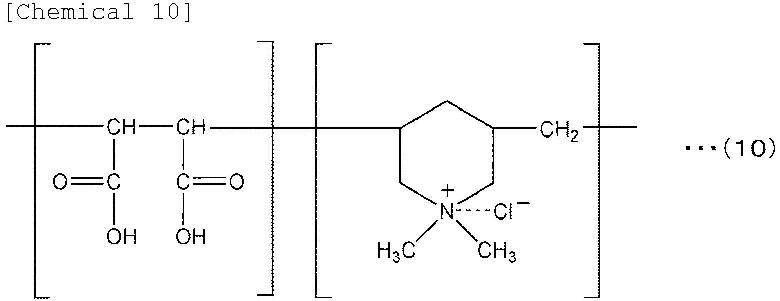

In addition, the polymer having a quaternary nitrogen may be a polymer having a group including a quaternary nitrogen in place of the six-membered ring including a quaternary nitrogen in the formulae (4) to (10). The polymer having a quaternary nitrogen may be a polymer having a five-membered ring including a quaternary nitrogen, a four-membered ring including a quaternary nitrogen, and a three-membered ring including a quaternary nitrogen in place of the six-membered ring including a quaternary nitrogen in the formulae (4) to (10).

In the blood collection container, the content of the heparin neutralizing agent is appropriately adjusted according to the type of the heparin neutralizing agent, the amount of blood collected, and the heparin concentration in the blood. In the blood collection container, the content of the heparin neutralizing agent is preferably 1×10⁻⁷ g or more, more preferably 5×10⁻⁶ g or more, still more preferably 1×10⁻⁵ g or more, and particularly preferably 1.5×10⁻⁵ g or more per 1 mL of collected blood. In the blood collection container, the content of the heparin neutralizing agent is preferably 1×10⁻¹ g or less, more preferably 1×10⁻² g or less, still more preferably 1×10⁻⁴ g or less, still more preferably 5×10⁻⁵ g or less, and particularly preferably 3.5×10⁻⁵ g or less per 1 mL of collected blood. When the content of the heparin neutralizing agent is equal to the above-described lower limit or more than the same, and equal to the above-described upper limit or less than the same, the effect of the present invention can be more effectively exhibited. In the blood collection container, the content of the heparin neutralizing agent is preferably 1×10⁻⁵ g or more and 5×10⁻⁵ g or less, and more preferably 1.5×10⁻⁵ g or more and 3.5×10⁻⁵ g or less per 1 mL of collected blood. In this case, the effect of the present invention can be particularly more effectively exhibited.

### (Inorganic powder: third region R3)

The blood collection container preferably includes an inorganic powder disposed in the blood collection container main body. The inorganic powder is preferably attached onto the inner wall surface of the blood collection container main body. In this case, the inorganic powder may be attached on the inner wall surface of the blood collection container main body via, for example, a water-soluble binder as stated below. One kind of the inorganic powder may be used alone, or two or more kinds thereof may be used in combination. The third region R3 is a region where the inorganic powder 3 is disposed.

Examples of the inorganic powder include silica powder, glass powder, kaolin powder, celite powder, and bentonite powder. One kind of the inorganic powder may be used alone, or two or more kinds thereof may be used in combination.

From the viewpoint of coagulating blood well, the inorganic powder is preferably silica powder, and more preferably porous silica powder.

The inorganic powder preferably has an average particle diameter of 1 mm or less, more preferably 100 µm or less, still more preferably 50 µm or less, and particularly preferably 10 µm or less. When the average particle diameter of the inorganic powder is the above-described upper limit or less, blood can be coagulated well in a short time.

The average particle diameter of the inorganic powder is an average diameter measured on a volume basis, and is a value of a median diameter (D50) corresponding to 50%. The volume average particle size (D50) can be measured by the laser diffraction/scattering method, the image analysis method, the Coulter method, the centrifugal sedimentation method, or the like. The volume average particle size (D50) is preferably determined by the measurement by the laser diffraction/scattering method or the image analysis method.

The inorganic powder has a linseed oil absorption capacity of preferably 20 ml/100g or more, and preferably 40 ml/100g or less. When the linseed oil absorption capacity of the inorganic powder is equal to the above-described lower limit or more than the same, and equal to the above-described upper limit or less than the same, the effect of the present invention can be more effectively exhibited.

The linseed oil absorption capacity of the inorganic powder is measured in accordance with JIS K-5101.

The inorganic powder has a BET specific surface area of preferably 5000 cm²/g or more and preferably 30000 cm²/g or less. When the BET specific surface area of the inorganic powder is equal to the above-described lower limit or more than the same, and equal to the above-described upper limit or less than the same, the effect of the present invention can be more effectively exhibited.

The BET specific surface area of the inorganic powder can be calculated by determining the amount of gas that, as a monolayer, can cover the surface of the inorganic powder, from the adsorption amount of gas adsorbed on the surface of the inorganic powder, the equilibrium pressure at that time, and the saturated vapor pressure of the adsorbed gas, and multiplying this by the average cross-sectional area of the adsorbed gas molecules. As the adsorption gas, nitrogen gas, oxygen gas, argon gas, methane gas, and the like can be used. In the BET specific surface area, the surface area including pores that cannot be measured by the measurement of the linseed oil absorption capacity can be measured.

In the blood collection container, the content of the inorganic powder is preferably 1×10⁻⁶ g or more, and more preferably 5×10⁻⁵ g or more, as well as preferably 1×10⁻² g or less, and more preferably 1×10⁻³ g or less per 1 mL of collected blood. When the content of the inorganic powder is equal to the above-described lower limit or more than the same, and equal to the above-described upper limit or less than the same, the blood can be coagulated well in a short time, and the effect of the present invention can be more effectively exhibited. Incidentally, when the content of the inorganic powder exceeds the above-described upper limit, this may affect the test result of serum.

In the first region, the serine protease may be disposed in the form of a layer, or may be formed in the form of islands such as dots. In the second region, the heparin neutralizing agent may be disposed in the form of a layer, or may be formed in the form of islands such as dots. In the third region, the inorganic powder may be disposed in the form of a layer, or may be formed in the form of islands such as dots.

A distance between the one end and the other end of the blood collection container main body is defined as L. The first region R1 is preferably present, in a direction from the one end toward the other end, in at least a part of a region extending from a position at 0.05 L to a position at 0.40 L, more preferably in at least a part of a region extending from a position at 0.06 L to a position at 0.35 L, further preferably in at least a part of a region extending from a position at 0.07 L to a position at 0.30 L, and particularly preferably in at least a part of a region extending from a position at 0.07 L to a position at 0.25 L. The first region R1 is preferably present, in a direction from the one end toward the other end, on the one end side with respect to a position at 0.40 L, more preferably present on the one end side with respect to a position at 0.35 L, further preferably present on the one end side with respect to a position at 0.30 L, and particularly preferably present on the one end side with respect to the position at 0.25 L. In this case, the effect of the present invention can be more effectively exhibited.

The second region R2 is preferably present, in a direction from the one end toward the other end, in at least a part of a region extending from a position at 0.40 L to a position at 0.90 L, more preferably in at least a part of a region extending from a position at 0.45 L to a position at 0.85 L, and further preferably in at least a part of a region extending from a position at 0.50 L to a position at 0.85 L. In this case, the effect of the present invention can be more effectively exhibited.

The third region R3 is preferably present, in a direction from the one end toward the other end, in at least a part of a region extending from a position at 0.01 L to a position at 1.00 L, more preferably in at least a part of a region extending from a position at 0.02 L to a position at 0.90 L, and further preferably in at least a part of a region extending from a position at 0.03 L to a position at 0.85 L. In this case, the effect of the present invention can be more effectively exhibited.

There is, or there is not, an overlapping region R12 where the first region R1 and the second region R2 overlap. There may be, or may not be, the overlapping region R12. From the viewpoint of more effectively exhibiting the effect of the present invention, it is preferable that there is not the overlapping region R12.

When there is the overlapping region R12, a ratio of a distance between the end on the one end side and the end on the other end side of the overlapping region R12 to a distance between the end on the one end side and the end on the other end side of the second region R2 (the distance between the end on the one end side and the end on the other end side of the overlapping region R12/the distance between the end on the one end side and the end on the other end side of the second region R2) is given as a ratio (R12/R2). The ratio (R12/R2) is preferably 0.2 or less, more preferably 0.1 or less, still more preferably 0.05 or less, and particularly preferably 0.01 or less. In this case, the effect of the present invention can be more effectively exhibited.

The distance between the end on the one end side and the end on the other end side of the overlapping region R12 and the distance between the end on the one end side and the end on the other end side of the second region R2 are distances in a direction of the one end and the other end of the blood collection container main body.

There is, or there is not, an overlapping region R13 where the first region R1 and the third region R3 overlap. There may be, or may not be, the overlapping region R13.

When there is the overlapping region R13, a ratio of a distance between the end on the one end side and the end on the other end side of the overlapping region R13 to a distance between the end on the one end side and the end on the other end side of the third region R3 (the distance between the end on the one end side and the end on the other end side of the overlapping region R13/the distance between the end on the one end side and the end on the other end side of the third region R3) is given as a ratio (R13/R3). The ratio (R13/R3) is preferably 0.40 or less, more preferably 0.35 or less, still more preferably 0.30 or less, and particularly preferably 0.25 or less. In this case, the effect of the present invention can be more effectively exhibited.

The distance between the end on the one end side and the end on the other end side of the overlapping region R13 and the distance between the end on the one end side and the end on the other end side of the third region R3 are distances in a direction connecting the one end and the other end of the blood collection container main body.

There is, or there is not, an overlapping region R23 where the second region R2 and the third region R3 overlap. There may be, or may not be, the overlapping region R23.

When there is the overlapping region R23, a ratio of a distance between the end on the one end side and the end on the other end side of the overlapping region R23 to a distance between the end on the one end side and the end on the other end side of the third region R3 (the distance between the end on the one end side and the end on the other end side of the overlapping region R23/the distance between the end on the one end side and the end on the other end side of the third region R3) is given as a ratio (R23/R3). The ratio (R23/R3) is preferably 0.6 or less, more preferably 0.3 or less, still more preferably 0.1 or less. In this case, the effect of the present invention can be more effectively exhibited.

The distance between the end on the one end side and the end on the other end side of the overlapping region R23 and the distance between the end on the one end side and the end on the other end side of the third region R3 are distances in a direction connecting the one end and the other end of the blood collection container main body.

### (Antifoaming agent)

The blood collection container preferably includes an antifoaming agent disposed in the blood collection container main body. The antifoaming agent is preferably attached onto the inner wall surface of the blood collection container main body. One kind of the antifoaming agent may be used alone, or two or more kinds thereof may be used in combination.

Examples of the antifoaming agent include polyoxyalkylene and polyoxyalkylene derivatives. Examples of the polyoxyalkylene derivative include polyoxyalkylene ethers.

Examples of the polyoxyalkylene ether include polyoxypropylene, polyoxypropylene glyceryl ether, polyoxyethylene, polyoxyethylene glyceryl ether, poly (oxyethylene-oxypropylene), and poly (oxyethylene-oxypropylene) glyceryl ether.

A region where the antifoaming agent is disposed is defined as a fourth region R4, and a distance between the one end and the other end of the blood collection container main body is defined as L.

The fourth region R4 is preferably present, in a direction from the one end toward the other end, in at least a part of a region extending from a position at 0.05 L to a position at 1.00 L, more preferably in at least a part of a region extending from a position at 0.07 L to a position at 0.90 L, and further preferably in at least a part of a region extending from a position at 0.10 L to a position at 0.85 L. The antifoaming agent is preferably disposed at least at the bottom of the blood collection container. In this case, when blood is collected, the blood comes into good contact with the antifoaming agent, and the occurrence of bubbles after the blood collection can be suppressed. As a result, the occurrence of blood clots involving bubbles can be more effectively suppressed.

Incidentally, there may be, or may not be, an overlapping region in which the fourth region R4 and the first region R1 overlap. There may be, or may not be, an overlapping region in which the fourth region R4 and the second region R2 overlap. There may be, or may not be, an overlapping region in which the fourth region R4 and the third region R3 overlap.

In the blood collection container, the content of the antifoaming agent is preferably 2.0×10⁻³ mg or more, and more preferably 3.0×10⁻³ mg or more, as well as preferably 0.2 mg or less, and more preferably 0.11 mg or less per 1 mL of collected blood. When the content of the antifoaming agent is equal to or more than the above-described lower limit, the antifoaming effect can be further enhanced. When the content of the antifoaming agent is equal to or less than the above-described upper limit, it is possible to suppress the occurrence of insoluble matters caused by the antifoaming agent, and thus it is possible to suppress the occurrence of troubles at the time of clinical examination such as clogging of the insoluble matters in the nozzle for sample collection at the time of clinical examination.

### (Serum separating composition)

The blood collection container preferably includes a serum separating composition housed at the bottom in the blood collection container main body.

As the serum separating composition, a conventionally known serum separating composition can be used. Examples of the serum separating composition include the serum separating composition described in WO 2011/105151A1 and the like.

The serum separating composition moves to between the serum layer and the blood clot layer to form a partition wall during centrifugation, to be used for the purpose of preventing component migration between the blood clot layer and the serum layer.

### (Other details of blood collection container)

The material of the blood collection container main body is not particularly limited. Examples of the material of the blood collection container main body include thermoplastic resins such as polyethylene, polypropylene, polystyrene, polyethylene terephthalate, polymethyl methacrylate, and polyacrylonitrile; thermosetting resins such as unsaturated polyester resins, epoxy resins, and epoxy-acrylate resins; modified natural resins such as cellulose acetate, cellulose propionate, ethyl cellulose, and ethyl chitin; silicate glasses such as soda lime glass, phosphosilicate glass, and borosilicate glass; and glasses such as quartz glass. As the material of the blood collection container main body, one kind may be used alone, or two or more kinds may be used in combination.

The blood collection container preferably includes a plug. As the plug, a conventionally known plug can be used. The plug is preferably formed of a such material in such a shape that the plug can be airtightly and liquid-tightly attached to the opening of the blood collection container main body. The plug is preferably configured such that a blood sampling needle can be inserted therethrough.

Examples of the plug include a plug having a shape fitted to the opening of the blood collection container main body, a sheet-like seal plug, and the like.

The plug may include a plug main body such as a rubber plug and a cap member made of plastic or the like. In this case, it is possible to suppress the risk that the blood comes into contact with a human body when the plug is pulled out from the opening of the blood collection container main body after the blood collection.

Examples of the material of the plug (or the plug main body) include synthetic resins, elastomers, rubbers, and metal foils. Examples of the rubber include butyl rubber and halogenated butyl rubber. Examples of the metal foil include an aluminum foil. From the viewpoint of enhancing the sealing property, the material of the plug (or the plug main body) is preferably butyl rubber. The plug (or the plug main body) is preferably a butyl rubber plug.

The blood collection container main body is preferably a blood collection tube main body. The blood collection container is preferably a blood collection tube.

The blood collection container may have an internal pressure that is not particularly limited. The blood collection container may be a vacuum blood collection container (vacuum blood collection tube) sealed by the plug after the inside is evacuated. When the blood collection container is a vacuum blood collection tube, it is possible to easily collect a certain amount of blood regardless of the technical difference of the blood collector.

From the viewpoint of preventing bacterial infection, the inside of the blood collection container is preferably sterilized in accordance with the standards described in ISO and JIS.

The blood collection container can be produced by disposing a first composition containing a serine protease and a second composition containing a heparin neutralizing agent, as well as a third composition containing an inorganic powder and a fourth composition containing an antifoaming agent as necessary, on the inner wall surface of the blood collection container main body. As a method for disposing them, known techniques such as coating, spraying, and attaching to the inner wall can be used.

The region where the first composition is disposed corresponds to the first region R1 in the blood collection container. The region where the second composition is disposed corresponds to the second region R2 in the blood collection container. The region where the third composition is disposed corresponds to the third region R3 in the blood collection container. The region where the fourth composition is disposed corresponds to the fourth region R4 in the blood collection container. A region where both the first composition and the second composition are disposed so as to overlap corresponds to the overlapping region R12 in the blood collection container. A region where both the first composition and the third composition are disposed so as to overlap corresponds to the overlapping region R13 in the blood collection container. A region where both the second composition and the third composition are disposed so as to overlap corresponds to the overlapping region R23 in the blood collection container.

The coating method is not particularly limited. Examples of the coating method include spray coating and dipping coating. It is preferable to apply each of the above-described compositions to the inner wall surface of the blood collection container main body and then dry the compositions.

In addition, it is preferable to manufacture the blood collection container by housing the serum separating composition before or after applying and drying each of the compositions, and attaching a plug to the opening of the blood collection container main body after applying and drying each of the compositions.

The application order (disposing order) of the first composition, the second composition, the third composition, and the fourth composition is not particularly limited. From the viewpoint of increasing the amount of the heparin neutralizing agent brought into contact with blood containing heparin, it is preferable to apply and dry the second composition after applying and drying the third composition and the fourth composition. It is preferable to apply and dry the second composition after applying and drying the first composition, the third composition, and the fourth composition.

Each of the first composition, the second composition, the third composition, and the fourth composition may contain other components such as water, an organic solvent, a water-soluble binder, an antifibrinolytic agent, an anti-plasmin agent, and a blood clot detachment component. In this case, each of the first region R1, the second region R2, the third region R3, and the fourth region R4 may contain the above-described other components.

Examples of the organic solvent include methanol, ethanol, butanol, isopropanol, and hexane. One kind of the organic solvent may be used alone, or two or more kinds thereof may be used in combination.

Examples of the water-soluble binder include polyvinyl alcohol, polyvinyl pyrrolidone, acrylic acid-based copolymers, and polyoxyalkylene block copolymers. One kind of the water-soluble binder may be used alone, or two or more kinds thereof may be used in combination.

Examples of the antifibrinolytic agent and the anti-plasmin agent include aprotinin, a soybean trypsin inhibitor, ε-aminocaproic acid, p-aminomethylbenzoic acid, and aminomethylcyclohexanecarboxylic acid. One kind of the antifibrinolytic agent and the anti-plasmin agent may be used alone, or two or more kinds thereof may be used in combination.

The antifibrinolytic agent and the anti-plasmin agent are preferably used in a clinically recommended amount. For example, it is preferable that aprotinin is used in an amount of about 100 KIU to 600 KIU (unit) per 1 mL of collected blood. The soybean trypsin inhibitor is preferably used, for example, in an amount of about 500 IU to 4000 IU (unit) per 1 ml of collected blood. Each of ε-aminocaproic acid, p-aminomethylbenzoic acid, and aminomethylcyclohexanecarboxylic acid is preferably used in an amount of, for example, about 10⁻⁸ g to 10⁻² g per 1 ml of collected blood.

Examples of the blood clot detachment component include silicone oil, polyvinyl pyrrolidone, polyvinyl alcohol, polyoxyalkylene, and derivatives thereof. One kind of the blood clot detachment component may be used alone, or two or more kinds thereof may be used in combination. From the viewpoint of effectively suppressing adhesion of blood to the inner wall surface or the like of the blood collection container, it is preferable that silicone oil, polyvinyl pyrrolidone, polyvinyl alcohol, polyoxyalkylene, or a derivative thereof is disposed as the blood clot detachment component in the blood collection container main body. Examples of the silicone oil include water-soluble modified silicone oils, which are preferably used. Examples of polyoxyalkylene and derivatives thereof described above include polyoxypropylene butyl ether, polyoxyethylene butyl ether, polyoxypropylene glyceryl ether, and polyoxyethylene glyceryl ether, which are preferably used. Polyoxypropylene glyceryl ether is a component that is also equivalent to an antifoaming agent.

Hereinafter, the present invention will be described in more detail with reference to Examples. The present invention is not limited only to the following examples.

A blood collection container main body as described below was prepared.

Blood collection container main body having the shape illustrated in Fig. 1

Inner diameter 10.8 mm × length 100 mm (length: distance between one end (open end) and the other end)
Material: polyethylene terephthalate

A plug described below was prepared.

Plug having a shape illustrated in Fig. 1
Rubber plug (material: butyl rubber)

The following serum separating composition was prepared.

Serum separating composition (thixotropic separating agent, available from Sekisui Chemical Co., Ltd.)

Materials of the first composition, the second composition, the third composition, and the fourth composition were prepared as shown below.

### (Serine protease)

Thrombin ("Thrombin Ito", available from Ito Pharmaceutical Co., Ltd.)

### (Heparin neutralizing agent)

Polyamine sulfone (5) (polycation having a structure represented by the formula (5)) ("PAS-H-5L" available from Nitto Boseki Co., Ltd.)
Polyamine sulfone (6) (polycation having a structure represented by the formula (6)) ("PAS-A-5" available from Nitto Boseki Co., Ltd.)
Polyamine sulfone (7) (polycation having a structure represented by the formula (7)) ("PAS-J-81" available from Nitto Boseki Co., Ltd.)
Polyamine sulfone (8) (polycation having a structure represented by the formula (8)) ("PAS-2351" available from Nitto Boseki Co., Ltd.)

### (Inorganic powder)

Silica powder (available from Unimin Specialty Minerals Inc., average particle diameter: 5 µm)

### (Antifoaming agent)

Polyoxypropylene glyceryl ether ("ADELA POLYETHER G-4000" available from ADEKA Corporation)

### (Water-soluble binder)

Polyvinylpyrrolidone ("PVP-K30" available from Wako Pure Chemical Industries, Ltd.)

The first composition was prepared as follows.

The first composition was obtained by completely dissolving 175000 units of thrombin, 0.1 g of polyvinylpyrrolidone and 0.6 g of β-alanine in 10 g of water.

The second compositions A to E were prepared as follows.

### Preparation of second composition A:

The second composition A was obtained by completely dissolving 0.36 g of the heparin neutralizing agent (polyamine sulfone (6)) and 0.8 g of polyvinylpyrrolidone in 100 g of water.

### Preparation of second composition B:

The second composition B was obtained by completely dissolving 0.4 g of the heparin neutralizing agent (polyamine sulfone (6)) and 0.8 g of polyvinylpyrrolidone in 50 g of water.

### Preparation of second composition C:

The second composition C was obtained by completely dissolving 0.36 g of the heparin neutralizing agent (polyamine sulfone (5)) and 0.8 g of polyvinylpyrrolidone in 100 g of water.

### Preparation of second composition D:

The second composition D was obtained by completely dissolving 0.36 g of the heparin neutralizing agent (polyamine sulfone (7)) and 0.8 g of polyvinylpyrrolidone in 100 g of water.

### Preparation of second composition E:

The second composition E was obtained by completely dissolving 0.36 g of the heparin neutralizing agent (polyamine sulfone (8)) and 0.8 g of polyvinylpyrrolidone in 100 g of water.

The third composition was prepared as follows.

The third composition was obtained by mixing 2.5 g of silica powder and 1.5 g of polyvinylpyrrolidone in 100 g of water.

The fourth composition was prepared as follows.

The fourth composition was obtained by mixing 0.3 g of polyoxypropylene glyceryl ether in 100 g of water.

The composition X was prepared as follows.

The composition X was obtained by completely dissolving 240000 units of thrombin, 0.36 g of the heparin neutralizing agent (polyamine sulfone (6)), 0.8 g of polyvinylpyrrolidone, and 1.2 g of β-alanine in 100 g of water.

### (Example 1)

In the bottom portion of the blood collection container main body, 0.9 g of the serum separating composition was housed. Subsequently, the second composition shown in Table 1 was spray-applied to the inner wall surface of the blood collection container main body, over the region shown in Table 1, and then dried. The first composition was then spray-applied over the region shown in Table 1, and then dried. Next, the pressure applied to the blood collection container main body was reduced to 13 kPa, and the blood collection container was sealed with a plug so that a blood collection container (vacuum blood collection tube) was produced.

### (Example 2)

In the bottom portion of the blood collection container main body, 0.9 g of the serum separating composition was housed. Subsequently, the fourth composition was spray-applied to the inner wall surface of the blood collection container main body, over the region shown in Table 1, and then dried. The second composition shown in Table 1 was then spray-applied over the region shown in Table 1, and then dried. The first composition was then spray-applied over the region shown in Table 1, and then dried. The same steps as those in Example 1 except for these were carried out, whereby a blood collection container (vacuum blood collection tube) was prepared.

### (Example 3)

A blood collection container (vacuum blood collection tube) was prepared in the same manner as in Example 1 except that the type of the second composition was changed as shown in Table 1.

### (Example 4)

The third composition was spray-applied to the inner wall surface of the blood collection container main body, over the region shown in Table 1, and then dried. The fourth composition was then spray-applied over the region shown in Table 1, and then dried. The second composition shown in Table 1 was then spray-applied over the region shown in Table 1, and then dried. The first composition was then spray-applied over the region shown in Table 1, and then dried. The same steps as those in Example 1 except for these were carried out, whereby a blood collection container (vacuum blood collection tube) was prepared.

### (Examples 5 to 8)

Blood collection containers (vacuum blood collection tubes) were produced in the same manner as in Example 4 except that the type of the second composition was changed as shown in Table 1.

### (Example 9)

The third composition was spray-applied to the inner wall surface of the blood collection container main body, over the region shown in Table 1, and then dried. The fourth composition was then spray-applied over the region shown in Table 1, and then dried. The first composition was then spray-applied over the region shown in Table 1, and then dried. Subsequently, the second composition shown in Table 1 was spray-applied over the region shown in Table 1, and immediately dried before the first composition was diffused. The same steps as those in Example 1 except for these were carried out, whereby a blood collection container (vacuum blood collection tube) was prepared.

### (Examples 10 to 13)

A blood collection container (vacuum blood collection tube) was prepared in the same manner as in Example 9 except that the type of the second composition was changed as shown in Table 1 and the region to which the composition was applied was changed as shown in Table 1.

### (Example 14)

The third composition was spray-applied to the inner wall surface of the blood collection container main body, over the region shown in Table 1, and then dried. The fourth composition was then spray-applied over the region shown in Table 1, and then dried. Subsequently, the second composition shown in Table 1 was spray-applied over the region shown in Table 1, and immediately dried before the second composition was diffused. The first composition was then spray-applied over the region shown in Table 1, and then dried. The same steps as those in Example 1 except for these were carried out, whereby a blood collection container (vacuum blood collection tube) was prepared.

### (Comparative Example 1)

A blood collection container (vacuum blood collection tube) was prepared in the same manner as in Example 4 except that the region to which the composition was applied was changed as shown in Table 1.

### (Comparative Example 2)

The composition X was spray-applied to the inner wall surface of the blood collection container main body, over the region shown in Table 3, and then dried. The same steps as those in Example 1 except for these were carried out, whereby a blood collection container (vacuum blood collection tube) was prepared.

### (Evaluation)

### (1) Frequency of occurrence of blood clots involving bubbles and frequency of production of fibrin in serum

Blood adjusted so that the concentration of heparin was 1 unit/mL was prepared. This blood, 5 mL, was collected in a 10 mL syringe (needleless, "JS-S10C" available from JMS), then collected in the obtained blood collection container (vacuum blood collection tube) using a transfer device ("REF364880" available from Becton, Dickinson and Company), and mixed by inversion 5 times. Thereafter, the blood collection container was left to stand still for 20 minutes. The blood collection container was then centrifuged at 1500G for 10 minutes. The blood collection container after the centrifugation was observed with the naked eye, and the presence or absence of blood clots involving bubbles and the presence or absence of fibrin in the serum were determined according to the following criteria. Each evaluation was performed by collecting blood of six persons.

### (1-1) Frequency of occurrence of blood clots involving bubbles and sizes thereof

Six blood collection containers were observed so that whether or not blood clots involving bubbles occurred was observed. When blood clots involving bubbles occurred, the sizes of the blood clots involving bubbles were classified into the following levels 1 to 3, and the respective numbers of the containers were determined. When no blood clot involving bubbles occurred, the container was classified as the level 0.

Level 0: No blood clot involving bubbles occurs
Level 1: The maximum length of the blood clots involving bubbles is 1 cm or less
Level 2: The maximum length of the blood clots involving bubbles is more than 1 cm and 2 cm or less
Level 3: The maximum length of the blood clots involving bubbles is more than 2 cm

### (1-2) Frequency of production of fibrin in serum and sizes thereof

Six blood collection containers were observed so that whether or not the production of fibrin was seen in the serum was observed. When the production of fibrin was seen in serum, the sizes of the fibrin were classified into the following levels 1 to 3, and the respective numbers of the containers were determined. When no production of fibrin was observed in the serum, the container was classified as the level 0.

Level 0: No production of fibrin was observed in serum
Level 1: The maximum diameter of fibrin produced in serum is 1 cm or less.
Level 2: The maximum diameter of fibrin produced in serum is more than 1 cm and 2 cm or less
Level 3: The maximum diameter of fibrin produced in serum is more than 2 cm.

The configurations and the results are shown in Tables 1 to 4. Incidentally, in Table 1, for each configuration, the drawing that shows a similar positional relationship among the first region, the second region, and the third region is indicated. In Table 2, the distance is a distance in a direction from the one end to the other end of the blood collection container main body.

**[Table 1]**

| | Configuration of blood collection container | First composition | | Second composition | | | | Third composition | | Fourth composition | |
|---|---|---|---|---|---|---|---|---|---|---|---|
| | | Thrombin (unit/1 mL of blood) | Application region and first region *1 | Type | Heparin neutralizing agent (µg/1 mL of blood) | Water-soluble binder (µg/1 mL of blood) | Application region and second region *1 | Silica powder (µg/1 mL of blood) | Application region and third region *1 | Antifoaming agent (µg/1 mL of blood) | Application region and fourth region *1 |
| Example 1 | Fig. 3 | 11 | 0.1L-0.2L | A | 15.8 | 35.2 | 0.55L-0.85L | - | - | - | - |
| Example 2 | Fig. 3 | 11 | 0.1L-0.2L | A | 15.8 | 35.2 | 0.55L-0.85L | - | - | 13.2 | 0.05L-0.85L |
| Example 3 | Fig. 3 | 11 | 0.1L-0.2L | B | 31.6 | 70.4 | 0.55L-0.85L | - | - | - | - |
| Example 4 | Fig. 4 | 11 | 0.1L-0.2L | A | 15.8 | 35.2 | 0.55L-0.85L | 110 | 0.05L-0.85L | 13.2 | 0.05L-0.85L |
| Example 5 | Fig. 4 | 11 | 0.1L-0.2L | B | 31.6 | 70.4 | 0.55L-0.85L | 110 | 0.05L-0.85L | 13.2 | 0.05L-0.85L |
| Example 6 | Fig. 4 | 11 | 0.1L-0.2L | C | 15.8 | 35.2 | 0.55L-0.85L | 110 | 0.05L-0.85L | 13.2 | 0.05L-0.85L |
| Example 7 | Fig. 4 | 11 | 0.1L-0.2L | D | 15.8 | 35.2 | 0.55L-0.85L | 110 | 0.05L-0.85L | 13.2 | 0.05L-0.85L |
| Example 8 | Fig. 4 | 11 | 0.1L-0.2L | E | 15.8 | 35.2 | 0.55L-0.85L | 110 | 0.05L-0.85L | 13.2 | 0.05L-0.85L |
| Example 9 | Fig. 5 | 11 | 0.1L-0.2L | B | 31.6 | 70.4 | 0.05L-0.85L | 110 | 0.55L-0.85L | 13.2 | 0.55L-0.85L |
| Example 10 | Fig. 5 | 11 | 0.1L-0.25L | A | 15.8 | 35.2 | 0.05L-0.85L | 110 | 0.55L-0.85L | 13.2 | 0.55L-0.85L |
| Example 11 | Fig. 5 | 11 | 0.1L-0.2L | A | 15.8 | 35.2 | 0.05L-0.85L | 110 | 0.55L-0.85L | 13.2 | 0.55L-0.85L |
| Example 12 | Fig. 6 | 11 | 0.1L-0.2L | A | 15.8 | 35.2 | 0.15L-0.85L | 110 | 0.55L-0.85L | 13.2 | 0.55L-0.85L |
| Example 13 | Fig. 6 | 11 | 0.1L-0.2L | A | 15.8 | 35.2 | 0.19L-0.85L | 110 | 0.55L-0.85L | 13.2 | 0.55L-0.85L |
| Example 14 | Fig. 7 | 11 | 0.1L-0.2L | A | 15.8 | 35.2 | 0.70L-0.85L | 110 | 0.05L-0.75L | 13.2 | 0.05L-0.85L |
| Comp. Ex. 1 | - | 11 | 0.1L-0.2L | A | 15.8 | 35.2 | 0.1L-0.2L | 110 | 0.05L-0.85L | 13.2 | 0.05L-0.85L |
| *1: a range where the composition is applied and a range where the region is present when a distance from one end to the other end of the blood collection container main body is given as L, the position of the one end is given as 0 L, and the position of the other end is given as 1 L. | | | | | | | | | | | |

**[Table 2]**

| | Distance of first region R1 | Distance of second region R2 | Distance of third region R3 | Distance of overlapping region R12 | Distance of overlapping region R13 | Distance of third region R23 | Ratio (distance of overlapping region R12 / distance of second region R2) | Ratio (distance of overlapping region R13 / distance of third region R3) | Ratio (distance of overlapping region R23 / distance of third region R3) |
|---|---|---|---|---|---|---|---|---|---|
| Example 1 | 0.1L | 0.30L | - | - | - | - | - | - | - |
| Example 2 | 0.1L | 0.30L | - | - | - | - | - | - | - |
| Example 3 | 0.1L | 0.30L | - | - | - | - | - | - | - |
| Example 4 | 0.1L | 0.30L | 0.80L | - | 0.1L | 0.30L | - | 0.13 | 0.38 |
| Example 5 | 0.1L | 0.30L | 0.80L | - | 0.1L | 0.30L | - | 0.13 | 0.38 |
| Example 6 | 0.1L | 0.30L | 0.80L | - | 0.1L | 0.30L | - | 0.13 | 0.38 |
| Example 7 | 0.1L | 0.30L | 0.80L | - | 0.1L | 0.30L | - | 0.13 | 0.38 |
| Example 8 | 0.1L | 0.30L | 0.80L | - | 0.1L | 0.30L | - | 0.13 | 0.38 |
| Example 9 | 0.1L | 0.80L | 0.30L | 0.1L | - | 0.30L | 0.13 | - | 1.00 |
| Example 10 | 0.15L | 0.80L | 0.30L | 0.15L | - | 0.30L | 0.19 | - | 1.00 |
| Example 11 | 0.1L | 0.80L | 0.30L | 0.1L | - | 0.30L | 0.13 | - | 1.00 |
| Example 12 | 0.1L | 0.70L | 0.30L | 0.05L | - | 0.30L | 0.07 | - | 1.00 |
| Example 13 | 0.1L | 0.66L | 0.30L | 0.01L | - | 0.30L | 0.02 | - | 1.00 |
| Example 14 | 0.1L | 0.15L | 0.70L | - | 0.1L | 0.05L | - | 0.14 | 0.07 |
| Comp. Ex. 1 | 0.1L | 0.1L | 0.80L | 0.1L | 0.1L | 0.1L | 1.00 | 0.13 | 0.13 |

**[Table 3]**

| | Configuration of blood collection container | Composition X | | |
|---|---|---|---|---|
| | | Thrombin (unit/1 mL of blood) | Heparin neutralizing agent (µg/1 mL of blood) | Application region *1 |
| Comp. Ex. 2 | - | 11 | 15.8 | 0.05L-0.85L |
| *1:a range where the composition is applied and a range where the region is present when a distance from one end to the other end of the blood collection container main body is given as L, the position of the one end is given as 0 L, and the position of the other end is given as 1 L. | | | | |

**[Table 4]**

| | Frequency of occurrence of blood clot involving bubbles and size thereof | | | | Frequency of production of fibrin in serum and size thereof | | | |
|---|---|---|---|---|---|---|---|---|
| | Level 0 (no occurrence) | Level 1 | Level 2 | Level 3 | Level 0 (no production) | Level 1 | Level 2 | Level 3 |
| Example 1 | 3 pieces | 3 pieces | 0 piece | 0 piece | 2 pieces | 0 piece | 4 pieces | 0 piece |
| Example 2 | 3 pieces | 3 pieces | 0 piece | 0 piece | 2 pieces | 0 piece | 4 pieces | 0 piece |
| Example 3 | 4 pieces | 2 pieces | 0 piece | 0 piece | 6 pieces | 0 piece | 0 piece | 0 piece |
| Example 4 | 6 pieces | 0 piece | 0 piece | 0 piece | 3 pieces | 3 pieces | 0 piece | 0 piece |
| Example 5 | 6 pieces | 0 piece | 0 piece | 0 piece | 6 pieces | 0 piece | 0 piece | 0 piece |
| Example 6 | 6 pieces | 0 piece | 0 piece | 0 piece | 3 pieces | 3 pieces | 0 piece | 0 piece |
| Example 7 | 6 pieces | 0 piece | 0 piece | 0 piece | 3 pieces | 3 pieces | 0 piece | 0 piece |
| Example 8 | 6 pieces | 0 piece | 0 piece | 0 piece | 3 pieces | 3 pieces | 0 piece | 0 piece |
| Example 9 | 6 pieces | 0 piece | 0 piece | 0 piece | 6 pieces | 0 piece | 0 piece | 0 piece |
| Example 10 | 4 pieces | 2 pieces | 0 piece | 0 piece | 0 piece | 0 piece | 6 pieces | 0 piece |
| Example 11 | 5 pieces | 1 piece | 0 piece | 0 piece | 0 piece | 0 piece | 6 pieces | 0 piece |
| Example 12 | 5 pieces | 1 piece | 0 piece | 0 piece | 2 pieces | 0 piece | 4 pieces | 0 piece |
| Example 13 | 6 pieces | 0 piece | 0 piece | 0 piece | 4 pieces | 0 piece | 2 pieces | 0 piece |
| Example 14 | 6 pieces | 0 piece | 0 piece | 0 piece | 5 pieces | 1 piece | 0 piece | 0 piece |
| Comp. Ex. 1 | 0 piece | 0 piece | 0 piece | 6 pieces | 0 piece | 0 piece | 0 piece | 6 pieces |
| Comp. Ex. 2 | 0 piece | 0 piece | 0 piece | 6 pieces | 6 pieces | 0 piece | 0 piece | 0 piece |

### EXPLANATION OF SYMBOLS

1, 1A, 1B, 1C, 1D, 1E, 1F: Serine protease
1a, 1Aa, 1Ba, 1Ca, 1Da, 1Ea, 1Fa, 1b, 1Ab, 1Bb, 1Cb, 1Db, 1Eb, 1Fb: End
2, 2A, 2B, 2C, 2D, 2E, 2F: Heparin neutralizing agent
2a, 2Aa, 2Ba, 2Ca, 2Da, 2Ea, 2Fa, 2b, 2Ab, 2Bb, 2Cb, 2Db, 2Eb, 2Fb: End
3, 3A, 3C, 3D, 3E, 3F: Inorganic powder
3a, 3Aa, 3Ca, 3Da, 3Ea, 3Fa, 3b, 3Ab, 3Cb, 3Db, 3Eb, 3Fb: End
4: Blood collection container main body
4a: One end
4b: Other end
5: Serum separating composition
6: Plug
11, 11A, 11B, 11C, 11D, 11E, 11F: Blood collection container
R1: First region
R2: Second region
R3: Third region
R12, R13, R23: Overlapping region

## Claims

1. A blood collection container comprising:
a blood collection container main body having an opening at one end thereof and a closed bottom at the other end thereof;
a serine protease disposed in the blood collection container main body; and
a heparin neutralizing agent disposed in the blood collection container main body,
wherein when a region in which the serine protease is disposed is defined as a first region and a region in which the heparin neutralizing agent is disposed is defined as a second region, the second region includes a region present on a side of the other end of the blood collection container main body, i.e., on the other end side, with respect to an end on the other end side of the first region.

2. The blood collection container according to claim 1,
wherein there is, or there is not, an overlapping region in which the first region and the second region overlap,
wherein when there is an overlapping region in which the first region and the second region overlap, the overlapping region having an end on a side of the one end of the blood collection container main body, i.e., on the one end side, and an end on the other end side, a ratio of a distance between the end on the one end side and the end on the other end side of the overlapping region to a distance between the end on the one end side and the end on the other end side of the second region is 0.2 or less.

3. The blood collection container according to claim 1 or 2, further comprising an inorganic powder disposed in the blood collection container main body,
wherein when a region in which the inorganic powder is disposed is defined as a third region, the third region includes a region present on the other end side with respect to an end on the other end side of the first region.

4. The blood collection container according to claim 3,
the third region including a region present on the one end side with respect to an end on the one end side of the second region.

5. The blood collection container according to claim 3 or 4,
the second region including a region present on the other end side with respect to an end on the other end side of the third region.

6. The blood collection container according to any one of claims 3 to 5,
the inorganic powder being silica powder.

7. The blood collection container according to any one of claims 1 to 6,
the serine protease being thrombin, a thrombin-like enzyme, or a fibrinogen degrading enzyme.

8. The blood collection container according to any one of claims 1 to 7, further comprising an antifoaming agent disposed at least at the bottom in the blood collection container main body.

9. The blood collection container according to any one of claims 1 to 8, further comprising a serum separating composition housed at the bottom in the blood collection container main body.
